# EUROPEAN PATENT APPLICATION

(11) **EP 1 297 781 A1**
(43) Date of publication of application: **02.04.2003**
(21) Application number: 02021634.7
(22) Date of filing: 27.09.2002
(51) Int. Cl.: A61B 5/00, A61B 5/103

(54) **Early detection of beauty treatment progress**

(30) Priority: 01.10.2001 US 325559; 21.12.2001 US 24619
(71) Applicant: L'OREAL, 75008 Paris (FR)
(72) Inventor: Pruche, Francis, 60300 Senlis (FR); Rubinstenn, Gilles, 75005 Paris (FR)
(74) Representative: Tanty, François

(57) **Abstract**

A method of tracking progress of a beauty treatment regimen is disclosed. A typical individual undergoing the beauty treatment regimen may exhibit at least one indicia of progress. The indicia of progress may be substantially imperceptible to un-enhanced sensory faculties, such as vision and touch, of a typical human in at least one stage of the beauty treatment regimen. The method may include prescribing a beauty treatment regimen to the individual, tracking the indicia of progress with respect to the individual, and advising the individual about positive progress in the beauty treatment regimen.

## Description

The present invention involves methods, combinations, apparatus, systems, and articles of manufacture for gauging the progress of a beauty treatment regimen. The invention may have particular use with treatments where initial indicia of progress are subtle or are otherwise imperceptible to a typical user.

The international patent application WO 00/76398 discloses an apparatus and method that enable to generate and to display a simulation showing improvement and/or worsening to the defect areas of a digital image of human skin.

However, this method does not enable to highlight substantially imperceptible indicia of progress.

Many beauty products require sustained use for a period of time before results are perceptible to a typical user. As a result, beauty product users may prematurely cease use of an effective treatment. The cessation may occur, for example, after an anti-wrinkle treatment has subtly diminished an appearance of wrinkles on the user's facial skin, but before improvement is visibly perceptible to the user.

Even when a beauty product user perceives some modicum of improvement, the user may still prematurely terminate for other reasons, including impatience or a perception that the product is ineffective. For example, the rate of perceived improvement may be such that the user loses confidence in the treatment's capability to achieve a desired measure of success. Such a perception of limited effectiveness, coupled perhaps with user impatience, prevents beauty product users from realizing the benefits of beauty products. And from a beauty product provider's perspective, premature cessation of treatments is highly undesirable. It undermines brand loyalty and trust, and reduces sales volumes.

Of course, premature cessation is not a phenomenon specific to the use of anti-wrinkle treatments. Rather, the phenomenon may also occur with any use of treatments such as those for hair loss, teeth whitening, tooth and hair coloring, skin firmness and elasticity, skin lightening, whitening, skin blemishes and freckles, tanning, other aesthetic-related conditions of the user and other biological conditions of the user.

Methods, combinations, apparatus, systems, and articles of manufacture consistent with the features and principles of the present invention may track progress of a beauty treatment regimen using indicia of progress.

One exemplary aspect consistent with the present invention is a method of tracking progress of a beauty treatment regimen. A typical individual undergoing a beauty treatment regimen exhibits at least one indicia of progress. This indicia of progress may be substantially imperceptible to a typical human eye in at least one stage of the beauty treatment regimen. Thus, the method may involve prescribing the beauty treatment regimen to an individual, tracking the indicia of progress with respect to the individual, and advising the individual about positive progress in the beauty treatment regimen.

The beauty treatment prescribed may not be a therapeutical or medical treatment.

A second exemplary aspect consistent with the present invention may include encouraging the individual to continue the beauty treatment regimen regardless of whether the positive progress is substantially imperceptible to the typical human eye.

A third exemplary aspect consistent with the present invention may include methods and systems for altering the prescribed beauty treatment regimen, particularly when reference to the indicia of progress suggests that a product may not be sufficiently effective for a particular subject. Altering may include prescribing an additional treatment that might have a synergistic effect when used with the originally prescribed beauty treatment regimen. Altering may also include abandoning the original regimen in favor of another one.

A fourth exemplary aspect consistent with the present invention may include methods and systems for prescribing the beauty treatment regimen to an individual, tracking an indicia of progress with respect to the individual, capturing at least two images of portions of the individual's body using an image capture means, comparing at least two images of portions of the individual's body to ascertain treatment effectiveness, and advising the individual of information reflective of the treatment effectiveness. The comparing of at least two images may use image processing techniques.

A fifth exemplary aspect of the present invention may include a method for performing a skin analysis. The method may comprise altering a chemical level in a subject's body, prescribing a beauty treatment regimen to the subject, receiving measurements reflecting a period of time required for the chemical level to cross a threshold level, determining from at least the measured period of time, a projected effectiveness of the prescribed beauty treatment regimen, and advising the subject of the projected effectiveness before an actual effectiveness is perceptible to the subject. Altering the chemical level may include adjusting a catalyst or phosphate level at a skin surface of the subject.

Additional aspects of the invention are set forth in the description which follow, and in part are apparent from the description, or may be learned by practice of methods (e.g., prognostics), combinations, devices, systems, and articles of manufacture consistent with features of the present invention. The aspects of the invention may be realized and attained by means of the elements and combinations particularly pointed out in the appended claims. It is understood that both the foregoing description and the following detailed description are exemplary and explanatory only and are not restrictive of the invention as claimed.

The accompanying drawings, which are incorporated in and constitute a part of this specification, illustrate several aspects of the invention and together with the description, serve to explain principles of the invention. In the drawings,
Figure 1A illustrates an exemplary flowchart for tracking a progress of the beauty treatment regimen consistent with features and principles of the present invention;
Figure 1B illustrates a second exemplary flowchart for tracking progress of a beauty treatment regimen consistent with features and principles of the present invention;
Figure 1C illustrates a third exemplary flowchart for tracking progress of a beauty treatment regimen consistent with features and principles of the present invention;
Figure 2A illustrates an exemplary light reflectivity measurement structure consistent with features and principles of the present invention;
Figure 2B illustrates exemplary portions of an individual's facial skin identified using the structure of Figure 2A;
Figure 3A illustrates an exemplary captured image consistent with features and principles of the present invention;
Figure 3B illustrates a second exemplary captured image consistent with features and principles of the present invention;
Figure 3C illustrates an exemplary graph projecting treatment progress consistent with features and principles of the present invention;
Figure 3D illustrates an exemplary graph comparing trends of progress consistent with features and principles of the present invention;
Figure 4 illustrates an exemplary method for assigning coloration values consistent with features and principles of the present invention;
Figure 5 illustrates an exemplary graph of progress data points consistent with features and principles of the present invention;
Figure 6 illustrates an exemplary questionnaire consistent with features and principles of the present invention;
Figure 7 illustrates aspects of a hair condition embodiment consistent with features and principles of the present invention; and
Figure 8 illustrates an exemplary system for tracking progress of a beauty treatment regimen consistent with features and principles of the present invention.

Reference is now made in detail to embodiments consistent with the invention, examples of which are illustrated in the accompanying drawings. Wherever possible, the same reference numbers are used throughout the drawings to refer to the same or like parts.

The invention may include a method of tracking progress of a beauty treatment regimen. Beauty treatment regimens may involve the administration of products or services including one or more of ointments, creams, gels, sprays, supplement, ingesta, inhalants, soaps, shampoos, scrubs, rinses, washes, hygienic tools and instruments, make-up, cosmetics, physical therapies, exercises, routines, and any other aesthetic improvement or maintenance product, service, combination, device, system, or article of manufacture. Such products or services may be used to treat any aesthetic feature of an individual's body and/or skin conditions such as wrinkles, aging, elasticity, coloration, clarity, freckles, blemishes and/or any other skin condition. Such products or services may also be used to treat hair conditions such as keratin plug conditions, hair length, dryness, oiliness, dandruff, thickness, density, root conditions, split ends, hair loss, and/or any other hair condition. Such products or services may additionally treat nail conditions such as color, gloss, strength, brittleness, thickness, hangnail, length, disease, and/or any other nail condition.

By way of example, treatments for wrinkles may be aimed at decreasing the number, severity, and/or the appearance of wrinkles, and any other qualities associated with decreasing lines or creases in skin. Treatments for aging may be aimed at combating conditions such as liver spots, hair loss, yellowing of teeth, and other causes and symptoms of growing old. Treatments for elasticity may be aimed at decreasing skin sag and improving suppleness, stress, softness, springiness and firmness of skin. Treatments for coloration (tanning and lightening) may be aimed at lightening, darkening, coloring, highlighting, shading, tinting, tinging, and shadowing of skin. Treatments for clarity may be aimed at increasing cleanliness, translucency, clearness and luminosity and decreasing blotchiness and pore size of skin. Treatments for blemishes may be aimed at removing, reducing, or masking appearance of moles, birthmarks, acne, blackheads, whiteheads, pockmarks, warts, pustules, boils, blisters, marks, smudges, and specks in skin. And treatments for freckles may be aimed at removing, diminishing, or masking appearance of small brownish spots on the skin.

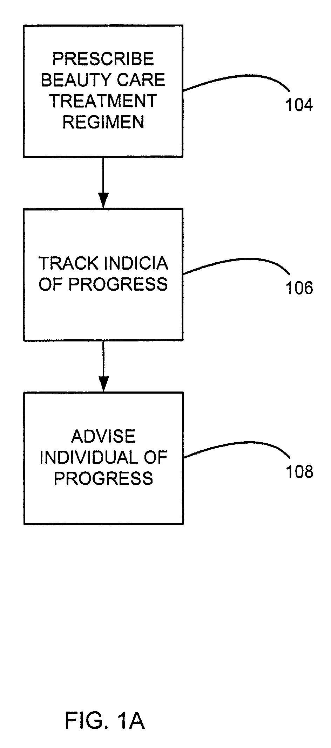

One exemplary method of tracking progress of a beauty treatment regimen is graphically illustrated in Figure 1A. Although the steps in Figure 1A (and other figures herein) are illustrated in an order, the invention, in its broadest sense, is not necessarily limited to a particular order. In addition, steps may be omitted and/or steps may be added without departing from the scope and spirit of the invention.

As shown at step 104, the method may involve prescribing a beauty treatment regimen to an individual. "Prescribing" may include providing an individual with advice regardless of form, suggesting either directly or indirectly to use one or more beauty products or services. Such a suggestion may occur in response to a specific request from the individual, or may be made in response to collecting individual-specific information provided through electronic or physical channels. The prescription may suggest use of a specific product(s), service(s), class of product(s), or service(s) and/or a product or service brand. The prescription may or may not contain application and use advice.

A typical individual undergoing the beauty treatment regimen may exhibit at least one indicia of progress. The indicia of progress may be substantially imperceptible to a typical human eye in at least one stage of the beauty treatment regimen. For example, if an individual's face contains forty-three discrete wrinkles, the elimination of four wrinkles may not be readily appreciable to the individual. Or if eight of the forty-three wrinkles lessen in intensity, with thirty-five remaining at the same intensity, such changes may not be readily appreciated. Thus, the term "substantially imperceptible to the human eye" is not intended to necessarily require that a human cannot, upon careful examination, note the progress. Rather, it means that the changes may be sufficiently slight that when looking in the mirror as part of an ordinary daily routine, the individual may be unlikely to perceive an improvement from a previous day. Or, it may mean that magnification may be required to detect changes in skin conditions that may not typically be seen. Magnification may be performed digitally on a computer or using analog methods such as zooming with an optical lens.

Alternatively, the indicia of progress might be totally invisible to the human eye. For example, chemical (e.g. catalysts, enzymes, etc.) levels in the skin can serve as early indicators of progress. With respect to an anti-wrinkle treatment, for example, enzymatic activity of phosphate in the stratum corneum may be measured as an early indicator of anti-wrinkle treatment efficacy. Since the recovery rate of the phosphate level may be a good indicator of anti-wrinkle treatment progress, a phosphate level at the surface of a subject's skin may be artificially altered (e.g. reduced through electromagnetic irradiation) at the beginning of an anti-wrinkle treatment. Thereafter, the phosphate level may be tracked and the subject advised of whether the treatment is likely to be successful. In this way, the subject may learn of likely success prior to significant signs of visible progress.

By way of another example, illuminating a portion of skin with a Woods lamp would reveal a location and density of p. acnes (microorganism associated with acne). The Woods lamp may help identify an amount of acne-causing bacteria on the portion of the subject's skin by making visible on the subject's skin residues, such as porphyrine, excreted by the bacteria. Thus, taking two pictures under a Woods lamp before and after treatment with a prophylactic and comparing the two pictures may reflect an efficacy of the treatment.

In yet another example, cosmetic powder with a special dye may be applied to the face to make visible the wrinkles. The cosmetic powder may highlight wrinkles by falling into crevices of folds in the wrinkles.

With respect to wrinkles, indicia of progress may include a change in wrinkle intensity (e.g. width, depth, density) and wrinkle frequency (e.g. number of wrinkles). Indicia of progress for conditions other than wrinkles may include changes in density of blemishes, number of blemishes, intensity of blemishes, blemish size, density of freckles, number of freckles, intensity of freckles, size of freckles, tone, coloration, pH level, moisture level, reflectivity, and characteristics of the individual that may correlate to a particular aesthetic condition of the individual.

The indicia of progress may be substantially imperceptible at the inception of a treatment regimen. However, in its broadest sense, the invention may be used at any time before, during, or after inception of a treatment regimen.

The invention may also include tracking indicia of progress with respect to the individual. This aspect is depicted at step 106 in Figure 1A. Tracking may include, but is not limited to, one or more of observing, marking, following, recording, graphing, measuring, calculating, projecting, and monitoring the indicia of progress. Tracking may be accomplished using a camera, scanner, web camera, test kit, or any other mechanism for gleaning progress data from the individual. If tracking involves the analysis of an image, image processing techniques and/or software may be used to identify and quantify the indicia of progress. Thus, continuing with the wrinkle treatment example, wrinkle severity information from a first image may be compared with wrinkle severity information from a second later image. If the processing reveals a change, the change may be quantified and compared with an expected improvement curve or indicator.

Techniques for processing images may involve binarizing the image to aid in locating conditions. Binarization may increase the contrast of the image to facilitate condition detection. Fourier transforms, fast Fourier transforms (FFTs), and/or discrete cosine transforms may be performed on all or part of the image, resulting in coefficients. Based on the coefficients, conditions may be located, as known in the art. Neural networks may also be used to identify conditions. Using the disclosure herein, persons skilled in the art would know how to train a neural network to accomplish these functions. Alternatively, one or more digital filters may be passed through the image for locating specific conditions. These examples are provided for illustrative purposes with the understanding that any image processing technique may be used within the scope and spirit of the invention.

The invention may also include advising the individual about positive progress in the beauty treatment regimen. This aspect is depicted at step 108 in Figure 1A. Advising may be accomplished through graphical displays, charts, text, audio presentation, video presentation, text message, or any other mechanism for conveying progress to the individual.

A determination as to whether positive progress has been achieved may be based upon a comparison with pre-stored information. The pre-stored information may be data relating to others who have followed the same or similar treatment regimen. Alternatively, the pre-stored information may be based on the experience of knowledgeable persons in the field. In any instance, there may be a baseline or threshold against which the individual's progress is gauged. If the individual meets or exceeds the baseline or threshold, the individual may be advised of positive progress.

For example, a goal of the beauty treatment regimen may be to decrease oiliness on portions of the individual's facial skin. The beauty treatment regimen may include the use of a lotion XYZ to decrease oiliness. A reflectivity index that correlates to oiliness may be used to track the progress of the beauty treatment regimen. A high reflectivity index may indicate substantial oiliness and a low reflectivity index may indicate insignificant oiliness. At a start of the beauty treatment regimen, the reflectivity index for portions of the individual's facial skin may be measured and recorded using a reflectivity measurement device.

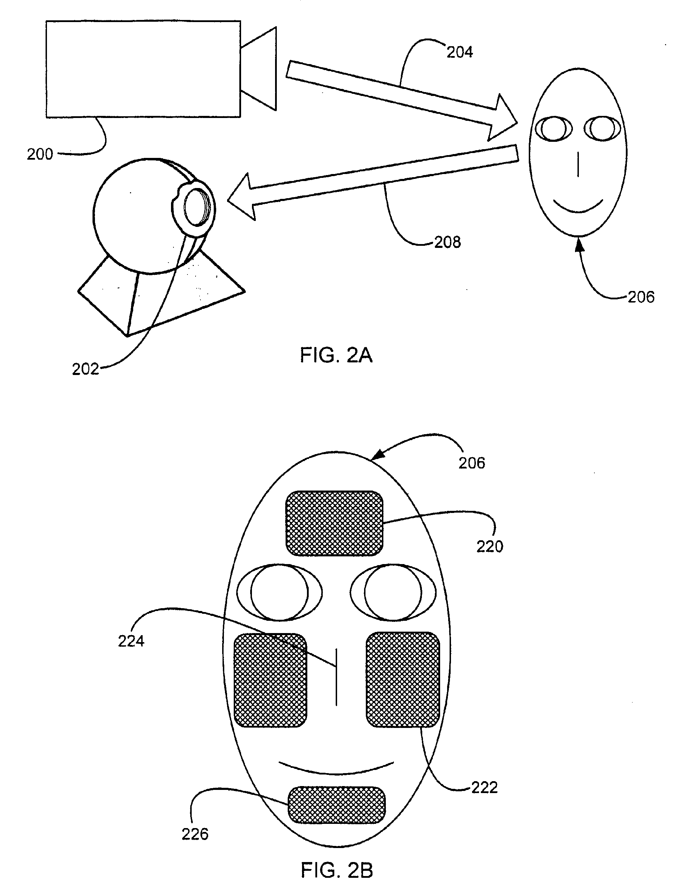

Figure 2A illustrates an exemplary light reflectivity measurement device consistent with features and principles of the present invention. The light reflectivity measurement device may include a light source 200 and a receptor 202. The reflectivity index may be measured by transmitting light 204 from the light source 200 onto the portions of the individual's facial skin 206 and receiving at receptor 202 light 208 reflected off the facial skin 206. If all transmitted light 204 is reflected and received by the receptor 202, then the reflectivity index may be one. If no transmitted light 204 is reflected and received by the receptor 202, then the reflectivity index may be zero. Reflectivity indices between zero and one indicate that transmitted light is partially reflected and received by the receptor. For example, a reflectivity index of one-quarter indicates that twenty-five percent of the transmitted light is reflected and received by the receptor. Portions of the individual's facial skin 206 that may have their reflectivity indices measured include, but are not limited to, forehead 220, cheeks 222, nose 224, and chin 226 as illustrated in Figure 2B.

After using the wash XYZ for two weeks, the individual may not notice any reduction of oiliness in portions of the individual's facial skin, but a second measurement of the reflectivity index may indicate subtle decreases in the reflectivity indices from the first measurement. The individual may then be advised about positive progress including the subtle decreases in the reflectivity indices which correlates to a decrease in oiliness on portions of the individual's facial skin.

A method consistent with the invention may also include encouraging the individual to continue with the beauty treatment regimen regardless of whether the positive progress is substantially imperceptible to a typical human eye. Encouragement may include presenting the positive progress to the individual as a reason to continue with the beauty treatment regimen, projecting the positive progress to forecast a time when the individual may expect to noticeably perceive progress, comparing the positive progress against trends of progress collected from other individuals, or otherwise conveying to the individual that progress is being made.

Positive progress may be determined using linear regression, mathematical models, statistics, or artificial intelligence. Artificial intelligence may include fuzzy logic, neural networks, genetic programming and decision tree programming. For example, positive progress may be compared against trends of progress using graphs, indices, charts, tables, or by altering a facial image to highlight for the individual changes that may have occurred. Such highlighting may involve image subtraction techniques, or other processing techniques, that illustrate only the changes that have occurred in the condition. Alternatively, the highlighting may involve visual cues added to the image such as coloration, shading, or other markings. Trends of progress may be calculated from or derivatives of demographic studies, surveys, empirical models, theoretical models, previous measurements of indicia of progress and other available information.

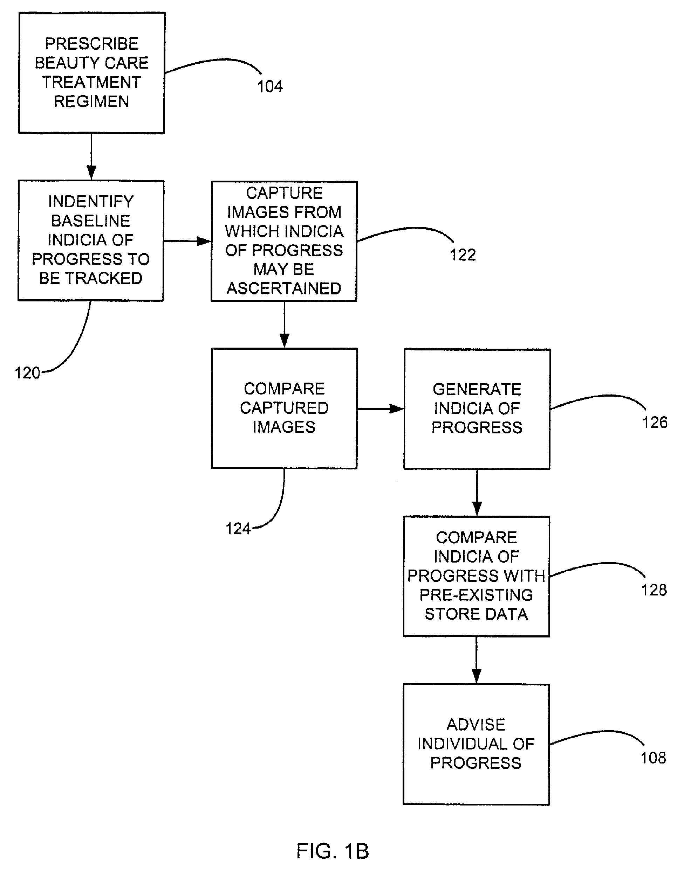

If indicia of progress is based upon a change detectable with an image capture device, the invention may be implemented in a manner consistent with the flow chart of Figure 1B. After a condition to be treated is identified and a prescription is made in step 104, a corresponding baseline characteristic of the individual is identified for tracking at step 120. An image capture device captures at least two time lapse images at step 122 and the time lapse images are compared at step 124. This may be accomplished through the image processing techniques, previously described. These image processing techniques may allow an indicia of progress to be generated at step 126 through, for example, a subtraction of the images. Once the indicia of progress is generated, it may be compared with known information to determine the significance of the progress as reflected at step 128. The known information may include collected demographic or survey data, or may be based upon the experiences of individuals in the field. After the significance of the indicia is determined, the individual may be advised of that significance as reflected in step 108.

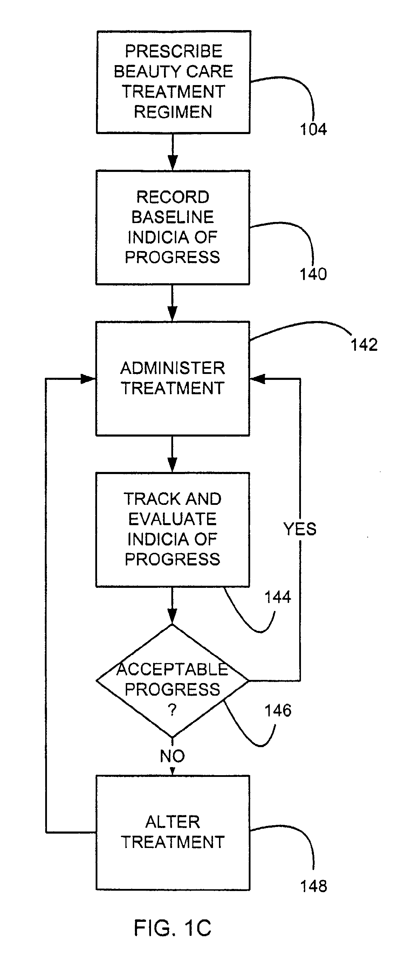

A further flowchart is presented in Figure 1C to reflect exemplary logic details that may be implemented in the invention. After prescription and recordation of a baseline at steps 104 and 140, respectively, the treatment may be administered at step 142. The treatment may be self-administered by the individual or administered with assistance from another individual (e.g. beauty professional). Tracking and evaluating of indicia of progress may be performed at step 144.

If the indicia of progress is below a desired threshold, then an alternation may be made in the treatment regimen. Examples of thresholds may include quantitative and qualitative levels against which the indicia of progress may be compared. The threshold may be used to determine whether a beauty treatment regimen has acceptable progress as illustrated at step 146. If the progress is not acceptable, the treatment regimen may be altered at step 148. The alteration may include adding an additional beauty treatment to the treatment regimen or discontinuing an existing beauty treatment in the treatment regimen. The additional beauty treatment may create a synergy as described later. The alteration may include abandoning the original beauty treatment regimen in favor of a different regimen.

By way of another example, a goal of the beauty treatment regimen may be to reduce the number of visible freckles in the individual's facial skin. An indicia of progress may be a reduction in number of freckles greater than a given size. Measurements of the indicia of progress may be performed by capturing an image of the individual's facial skin using an image capture device and counting the freckles using image processing techniques. While, in its broadest sense, the invention may not require an image capture device, image capture devices may be particularly well suited for implementing the invention. To this end, agents such as pigments may be used to provide skin contrast. Image capture devices may include web cameras, film cameras, analog cameras, digital cameras, scanners, infrared images, ultra-sound imaging devices or any other mechanism for acquiring a representation of the individual's facial skin.

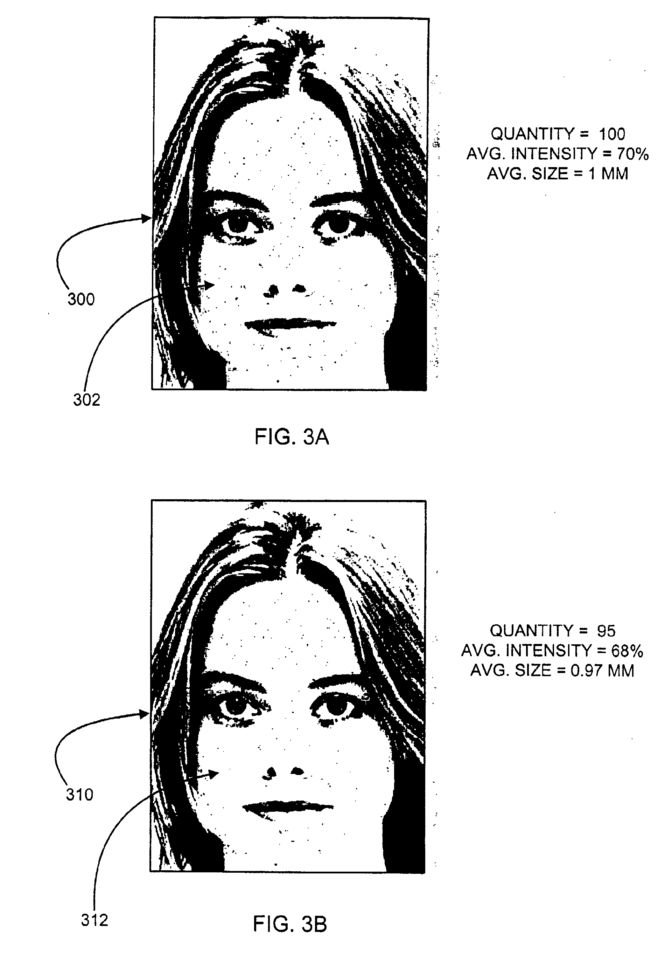

Figure 3A illustrates an exemplary captured image 300 of the individual's facial skin. An initial measurement from the captured image 300 may be one hundred freckles 302 on the individual's facial skin. The beauty treatment regimen may include a freckle reduction treatment. After one week of using the freckle reduction treatment, a second measurement from a second exemplary captured image 310, as illustrated in Figure 3B, may be ninety-five freckles 312. Thus the indicia of progress in this example is a freckle reduction of five percent.

Comparing a five percent reduction with an expected reduction after one week of treatment, it may be determined that the individual is on track for an effective end result. Nevertheless, from the individual's perspective, the positive progress may not yet be visibly perceptible. The individual may be encouraged to continue the beauty treatment regimen by advising the individual of the five percent reduction, and providing the individual with some basis to understand that a five percent reduction in one week is positive and is a predictor for a successful treatment. The individual may also be encouraged to continue the beauty treatment regimen by projecting from the measurements an expected time when the number of visible freckles may sufficiently decrease for the individual to perceive the difference.

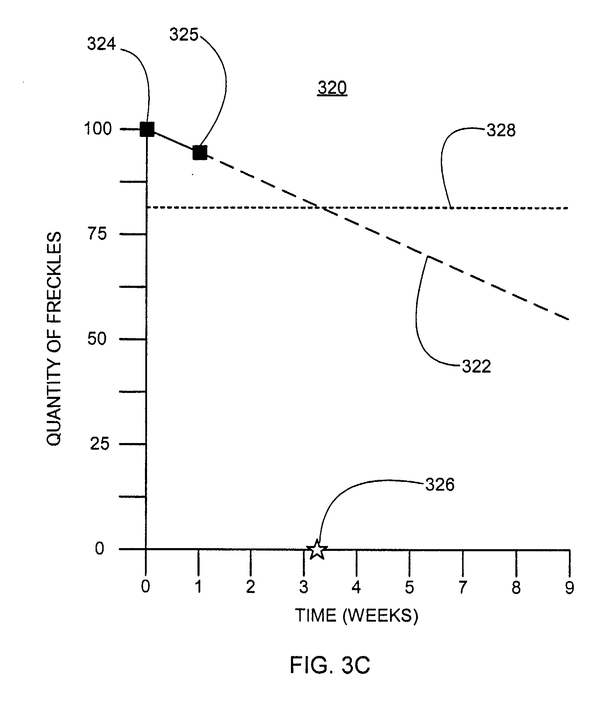

Figure 3C illustrates an exemplary graph 320 that uses multiple freckle measurements to project an expected time frame when treatment progress will be visibly perceptible. Graph 320 illustrates two actual measurements 324 and 325, respectively, taken at the inception of the treatment regimen and one week thereafter. From measurements 324 and 325, an expected wrinkle reduction curve 322 is generated. Threshold 328 defines a freckle reduction that should be readily perceptible to the individual. In graph 320, expectation curve 322 crosses the threshold 328 at time 326 just after the third week of treatment. By providing such information to the subject individual, the individual may be encouraged to continue with treatment regimen at least through the fourth week.

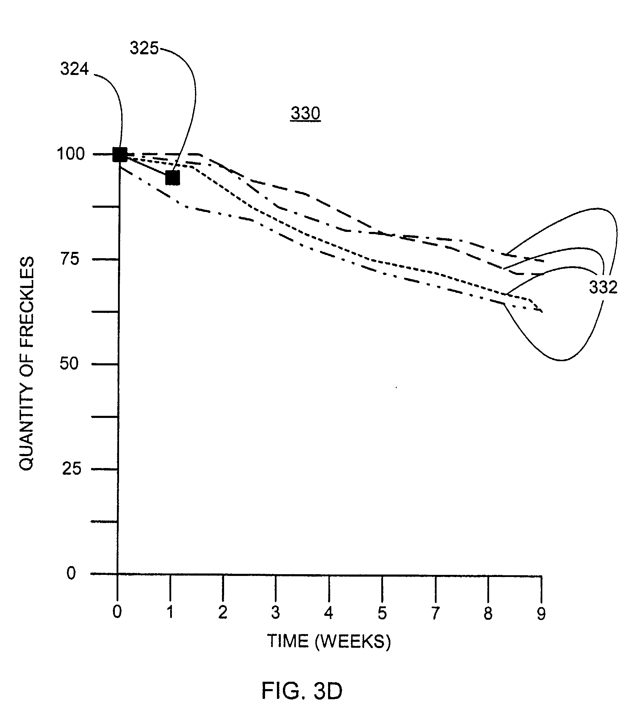

The subject individual may also be encouraged by comparing the positive progress against trends of progress of other individuals that may have used similar beauty treatment regimens. In Figure 3D, an exemplary graph 330 compares trends of progress of other individuals 332 against the subject's two measurements 324 and 325. The other individuals may be chosen for display due to similar personal characteristics to those of the subject. The trends of progress of other individuals may also typically display imperceptible positive progress at initial stages of the beauty treatment regimen, but the subject may be encouraged by a significant reduction in freckles for the other individuals at later stages, such as weeks three to nine.

A method consistent with the invention may also include advising the individual about an additional synergistic treatment for use with the prescribed beauty treatment regimen. After some initial data is gathered, it might be determined in an alteration of the original treatment regimen to add the use of one or more additional products might result in a synergistic effect. For example, a synergy might be achieved by combining a wrinkle treatment with an elasticity treatment.

If a goal of a beauty treatment regimen is to reduce dryness of the individual's skin, the originally prescribed regimen may include a beauty treatment for moisturizing the skin, such as skin lotion. Indicia of progress to track the effectiveness of the beauty treatment regimen may include measurements from a moisture measuring device applied to the individual's skin. An initial measurement may indicate low moisture in the individual's skin. After three days of undergoing the beauty treatment regimen, a second measurement of the indicia of progress may still indicate low moisture in the individual's skin. Since there is no significant difference between the first and second measurements, the indicia of progress indicates no reduction in dryness. An oral vitamin supplemental to the skin lotion may be advised to the individual. It may be known that the combination of the vitamin and lotion is likely to create a synergy that reduces dryness in the individual's skin. After a period of undergoing the beauty treatment regimen with the synergy, later measurements of the indicia of progress may indicate a increased moisture in the individual's skin.

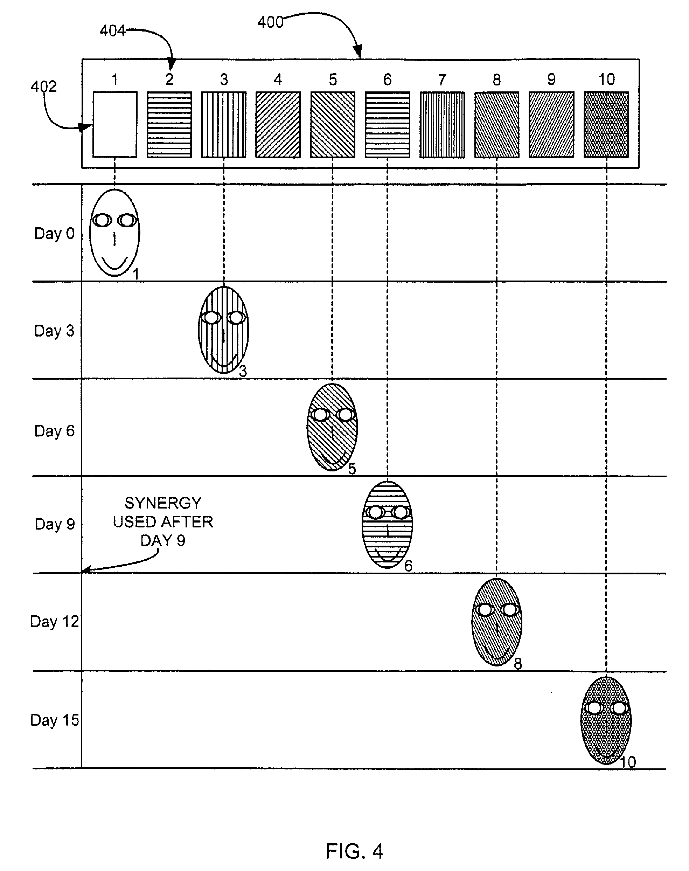

Advising the individual about a predicted synergy may occur when the indicia of progress is below a desired threshold. For example, a goal of the beauty treatment regimen may be to tan the skin. The beauty treatment regimen may require the individual to use an oral tanning product for short durations everyday over two weeks. Figure 4 illustrates how colorations of the individual may be assigned numeric coloration values. A coloration bar 400 may contain various color blocks 402 with corresponding coloration values 404 assigned to them. Higher coloration values indicate higher colorations. An individual's coloration may be compared against the color blocks 402 to determine the individual's coloration value. An indicia of progress may be a difference in coloration values. The difference represents an increase in coloration of portions of the individual's skin. Higher differences indicate greater increases in coloration and lower differences indicate lesser increases in coloration.

As exemplified in Figure 4, an initial coloration value of the individual on day zero is one, which indicates insignificant coloration. After undergoing the beauty treatment regimen for three days, the coloration value of the individual is three. An initial measurement for the indicia of progress may be a difference in coloration values between day zero and day three, which may equal two. The initial measurement of two may show a significant increase in coloration of the individual. A second measurement between day three and day six may also equal two, which may show another significant increase in coloration of the individual. However, a measurement between day six and day nine may equal one, which may be below a desired threshold of one-and-a-half. The individual may then be advised of a tanning lotion that tends to work well with the oral tanning product. The two beauty treatments may form a synergy and may be used in the beauty treatment regimen. Later measurements of the indicia of progress may be above the desired threshold.

Tracking the progress of the beauty treatment regimen may also include auto-evaluation by the individual. Auto-evaluation may include any method or means by which only the individual assesses the progress of her beauty treatment regimen. If, for example, the beauty treatment regimen is aimed at reducing blotchiness of the individual's facial skin, rather than using the digital camera to record the indicia of progress, tracking may require the individual to perform a task of using a magnifying mirror and counting and determining size and severity of blotches as seen from the mirror's reflection of the individual's facial skin. The individual may track the progress of the beauty treatment regimen by performing and recording results of the task several times over a period of using the beauty treatment regimen.

Tracking the progress of the beauty treatment regimen may also include directing the individual to conduct at least a portion of at least one self-test, and directing the individual to record a result of the at least a portion of at least one self-test. Self-tests may include test kits, meters, probes, and any other mechanism administered by the individual for evaluating the progress of the beauty treatment regimen. For example, a goal of the beauty treatment regimen may be to increase suppleness of the individual's skin. If there were to exist a correlation between surface pH levels and suppleness, the indicia of progress may be pH level of the individual's skin surface. This test could be self-administered by the individual.

A self-test including litmus paper for measuring pH levels of skin surfaces may be used to help track progress. Prior to starting the beauty treatment regimen, the individual may perform the self-test by applying the litmus paper to the individual's skin. The litmus paper may indicate results for a pH level of the individual's skin by turning red with increasing acidity or turning blue with increasing basicity. Over a course of the individual following the beauty treatment regimen, the individual may be directed to perform and record results of the self-test. If the self-test indicates that the pH levels are moving in a desired direction, then it may be concluded that the beauty treatment regimen is increasing the suppleness of the individual's skin. The increase in suppleness may be concluded even if the individual does not perceive the increase with her own un-enhanced senses.

Other than a pH level of the individual's skin, the self-test may alternatively include materials for checking elasticity, oiliness, texture, color, pigmentation of at least one of the individual's skin and hair, and any other characteristics that may correlate to various aesthetic-related qualities of the individual. Results of at least a portion of the self-test may be electronically reported during a beauty analysis. Examples of electronic reporting may include sending e-mail, manipulating web pages, making a phone call, conveying the results over the internet, and any mechanism for communicating results that involves electricity. Alternatively, used self-test materials may be transmitted to a laboratory for analysis. Results of the analysis may be reported to the individual.

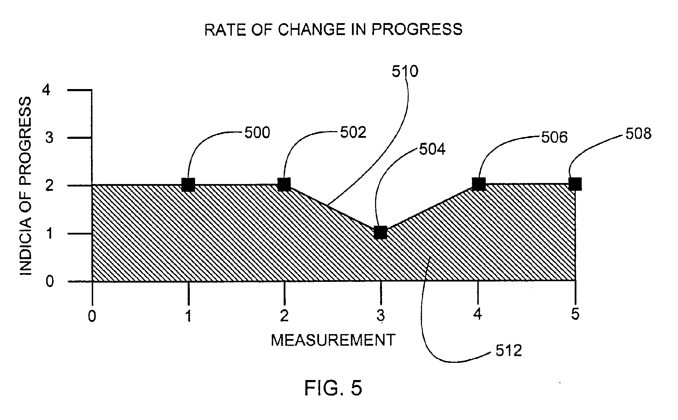

Tracking may also include collecting a series of data points to form a curve and calculating an area under the curve. The data points may be numeric representations of the indicia of progress. The area may be an alternative quantification of the progress of the beauty treatment regimen. In the above tanning example for increasing coloration in the individual as if from a sun tan, multiple indicia of progress derived from measurements on days three, six, nine, twelve, and fifteen may be reflected as data points 500-508, respectively, in an exemplary graph illustrated in Figure 5. The data points may form a curve 510 and an area 512 underneath the curve 510 may be calculated. In this example, the area 512 is nine, corresponding to a total increase of coloration value in the individual over fifteen days of the treatment.

Consistent with the invention, at least a portion of a beauty analysis may be prescribed over a network. Networks may include public networks such as the Internet, telephony networks, courier networks (e.g. postal service, United Parcel Service, Federal Express, etc.), private networks, virtual private networks, local area networks, metropolitan area networks, wide area networks, ad hoc networks, and any methods, combinations, devices, systems, and articles of manufacture for facilitating communication and/or conveyance of data between two locations, regardless of whether the connection is wired or wireless.

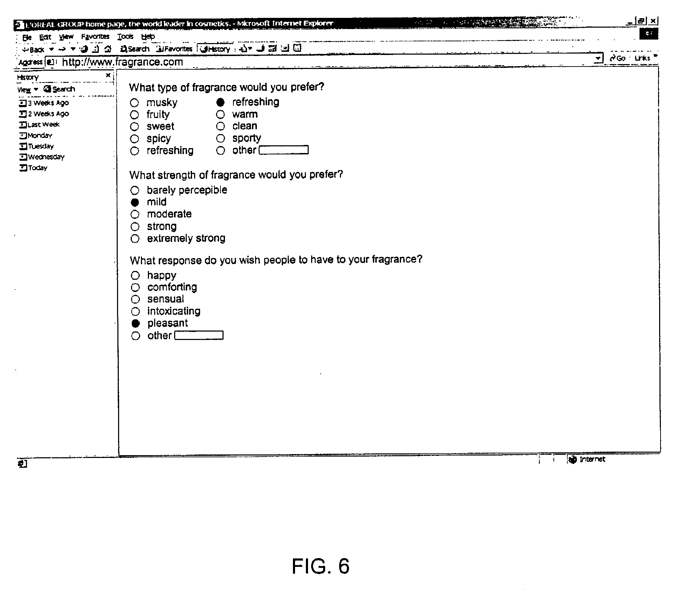

By way of another example, a goal of a beauty treatment regimen may be to increase the effectiveness of perfume for the individual. The individual may complete a web page questionnaire on a computer over the Internet, such as an exemplary questionnaire 600 illustrated in Figure 6. The questionnaire may be a beauty analysis that requests information from the individual such as desired type, strength, and response of other individuals to the scent of the individual. Types of scent may include musky, fruity, sweet, spicy, refreshing, warm, clean, sporty, and any other possibility. Strength of scent may include anything in a range from barely perceptible to extremely strong. The response of other individuals may include happy, comforting, sensual, intoxicating, pleasant, and any other desired feeling.

An analysis of the information provided by the individual in the questionnaire may produce suggestions for the beauty treatment regimen. The suggestions may prescribe a scent-neutralizing soap and perfume as beauty treatments in the beauty treatment regimen. Indicia of progress may include chemical residue of the perfume on the individual's skin. Mechanisms for measuring the chemical residue may be applied to the individual's skin to track progress.

Methods consistent with the invention may be performed with aid of a computer-related product. Examples of computer-related products may include software programs, computers, processors, personal digital assistants, and mobile communication devices. Computer-related products may also include web sites configured to enable the functionality of the invention or any other electronic mechanism for providing one or more of the functions of the invention. Activities that may be accomplished by a computer-related product may include encouraging the individual to continue the beauty treatment, advising the individual about synergies, prescribing and altering the beauty treatment regimen, capturing images, providing instructions to the individual, conducting beauty analyses, electronically reporting results of self-tests, and any other tasks consistent with principles of the invention. Electronic forms of the invention are not limited to a web browser implementation.

For example, a kiosk may be located at a cosmetics counter in a store facilitating beauty analysis. A computer in the kiosk may be configured to perform one or more of the prescribing, tracking, and advising activities.

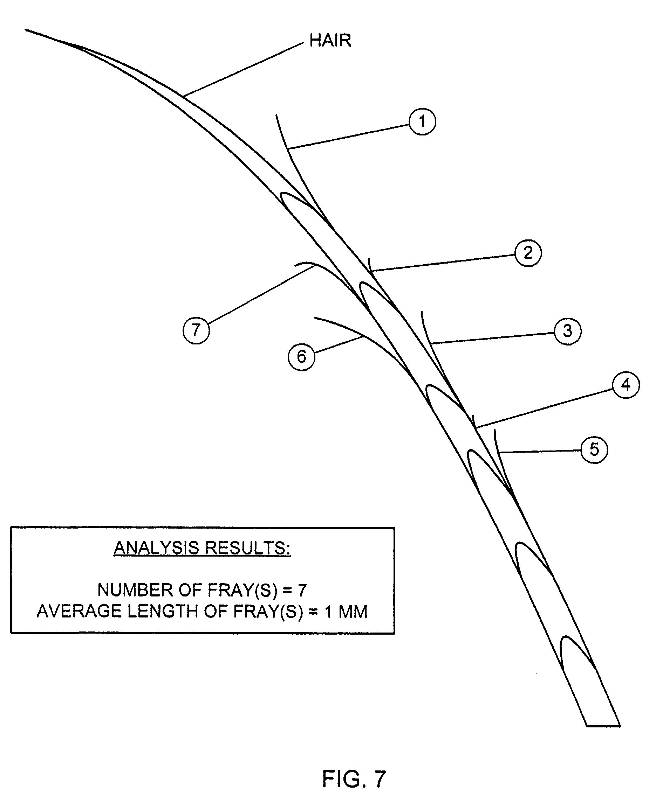

By way of yet another example, the individual may use the computer to perform a beauty analysis such that a beauty guidance on how to decrease the number of split ends in the individual's hair as depicted in Figure 7. The beauty treatment regimen may include the daily use of shampoo and conditioner beauty treatments. The indicia of progress may be a reduction in the number frays in sample hairs provided by the individual. Number of frays may be measured by capturing an image of the sample hairs using a digital camera in a kiosk or any other location. The computer may be configured to perform image processing on the image. Image processing may include magnifying the image, isolating individual hairs in the image of the sample hairs, and counting number of frays per hair. As with the other examples set forth herein, previously discussed image processing techniques may be employed to measure progress.

An initial measurement of conditions, such as number of frays, may be recorded by the computer. After five days of performing the beauty treatment regimen, the individual may not notice a decrease in split ends. The individual may then supply another hair sample to the computer for counting of a most recent number of frays. If the number of frays has decreased between the initial measurement and the most recent number of frays, then the computer may project an additional number of days to continue the beauty treatment regimen before the individual may notice a decrease in split ends. The computer may encourage the individual to continue the beauty treatment regimen in a manner consistent with those previously described.

In the kiosk example described above, a local process may perform all the portions of prescribing, tracking, and advising. Alternatively, the kiosk may be connected to a computer network that may perform some of the prescribing, tracking, and advising tasks. Within the computer network, there may exist a host computer that may perform the image processing in place of the kiosk process. Or, the network may include a physical courier network, such as the postal service. The individual may send samples of the individual's hair or results of a self-test on the hair by mail to a laboratory for image processing. Positive progress and encouragement to continue the beauty treatment regimen may be provided to the individual by correspondence over the postal network.

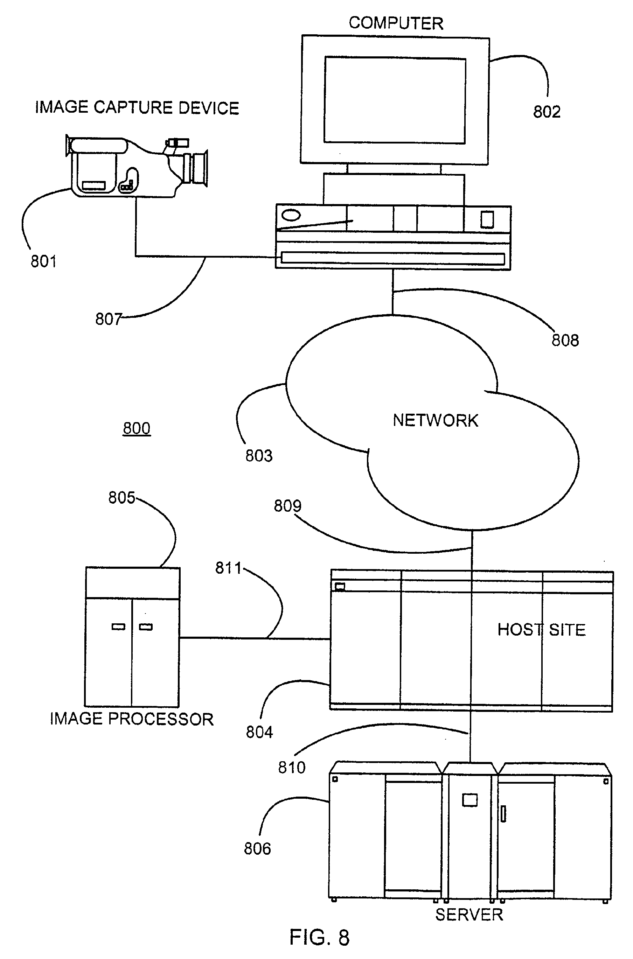

Figure 8 illustrates an exemplary system 800 for tracking progress of the beauty treatment regimen in which principles of the present invention may be implemented. The system 800 may include an image capture device 801, computer 802, network 803, host site 804, image processor 805, and server 806. Image capture device 801 and network 803 may include any of the previously described examples. Image capture device 801 may have a first communication link 807 to the computer 802. Computer 802 may have a second communication link 808 to the network 803. Network 803 may have a third communication link 809 to the host site 804. Host site 804 may have fourth and fifth communication links 810 and 811 to the image processor 805 and server 806, respectively.

The image capture device and computer may collect information about the individual currently participating or wishing to start participating in the beauty treatment regimen. Information may include images of portions of the individual's body to be used to track indicia of progress. Portions of the information may be conveyed over the network 803 to at least one of the host site 804, image processor 805, and server 806. Portions of the information may be image processed by the image processor. The image processing may include tests and analyses to identify, evaluate, and characterize the indicia of progress. Recommendations for beauty treatment regimens may be prescribed to the individual by at least one of computer 802, host site 804, image processor 805, and server 806.

Progress in the beauty treatment regimen may be tracked and evaluated by at least one of elements 801-806 in the system 800. At various times during participation in the beauty treatment regimen, the image capture device and computer may collect additional information about the individual. Portions of the additional information may be conveyed over the network 803 to at least one of the host site 804, image processor 805, and server 806. Portions of the additional information may be image processed by the image processor. The evaluation may include a comparison of the additional information with the information originally collected by the image capture device and computer. The evaluation may also include manipulating and comparing image processed information.

Tracked progress in the beauty treatment regimen may be characterized and conveyed to the individual. The tracked progress in the beauty treatment regimen may be conveyed by the system 800. The system 800 may make recommendations to the individual to continue the beauty treatment regimen or alter the beauty treatment regimen.

This application may discuss beauty products in connection with use by women. However, it is to be understood that such discussions are for exemplary purposes only. It is to be understood that the invention is equally applicable to all genders, and is not necessarily limited to the beauty industry. It is also to be understood that any functional aspect of the invention can be implemented via any location in the system or network, and data software may be resident at any location either in a network, at a stand-alone site, or on media in the custody and control of a user or subject.

It is to be further understood that the physical mechanisms (e.g. hardware, software, networks, systems) for implementing the methods of the invention are many. Networks, hardware and systems can be configured in a host of ways with software and hardware functionality residing at many alternative locations. In addition, systems other than the exemplary systems disclosed might be used to implement the invention. Therefore, it is to be understood that the methods of the invention are not limited to any particular structure.

Further, methods or portions thereof can be implemented in either an electronic environment, a physical environment, or combinations thereof. Thus, for example, although one or more portions of a method may occur in an electronic environment, a "purchase" portion of the method may occur in a brick and mortar store, or vice versa.

To the extent not inconsistent with the invention defined herein, the following global definitions are to be considered in interpreting the language of this patent and the claims herein. Where multiple definitions are provided, they should be considered as a single cumulative definition.

The term "image" may include one or more of two-dimensional and three-dimensional representations. In certain examples consistent with the invention, a plurality of images from different perspectives may be used to construct a three-dimensional image. In a broader sense, only a single image may be used. Depending on the embodiment, the term "image" may include either a visually perceptible image or electronic image data that may be either used to construct a visually perceptible image or to derive information about the subject. The image may be a body image corresponding to an anatomical portion of the subject, and may represent, for example, the subject's entire face, or a portion of the subject's face. The image may be a detailed picture (e.g., a digital image or a photograph) of a portion of the subject's body and/or a topological plot mapping contours of a portion of subject's body. If the image is representative of an external body condition, the image could be either an actual image showing the condition or an image including symbolizations of the condition, for example. The image may be an actual or a simulated image. Simulated images may include wholly or partially generated computer images, images based on existing images, and images based on stored features of a subject.

The term "image capture device", similar terms, and terms representing structures with similar functions may include one or more of a digital camera, webcam, film camera, analog camera, digital video camera, scanner, facsimile machine, copy machine, infrared imager, ultra-sound imaging device, or any other mechanism for acquiring an image of a subject's external body condition, an image of the subject's countenance, an/or an image of the subject's skin. An ultrasonic device might provide skin thickness information, or it might create a map on an area of the external location. Thus, the term "image" as used herein may be broader than a picture. Combinations of image capture devices may be used. For example, an image captured on photographic paper using a film camera might then be scanned on a flat bed scanner to create another image.

The term "capturing (an image)", or any form thereof, refers to the use of an image capture device to acquire an image. "Capturing" may refer to the direct act of using the image capture device to acquire the image. It may also include indirect acts to promote acquisition. To this end, "capturing" may include the indirect acts of providing access to hardware, or to at least one of a client-based algorithm and a server-based algorithm for causing the image capture device to capture an image. This may be accomplished by providing a user with software to aid in the image capture process, or providing the user with access to a network location at which the software resides. Also consistent with certain embodiments of the invention, capturing may include at least one of receiving an instruction from the subject to capture an image, indicating to the subject before the image is captured, and indicating to the subject when the image is captured.

The term "image processing technique" or similar terms, may include a software program, computer, application specific integrated circuit, electronic device and/or a processor designed to identify in an image one or more characteristics, such as a skin condition. Such techniques may involve binarization, image partitioning, Fourier transforms, fast Fourier transforms (FFTs), and/or discrete cosine transforms may be performed on all or part of the image, resulting in coefficients. Based on the coefficients, conditions may be located, as known in the art. Artificial intelligence, such as fuzzy logic, neural networks, genetic programming and decision tree programming, may also be used to identify conditions. Alternatively, one or more digital filters may be passed through the image for locating specific conditions. These examples are provided for illustrative purposes with the understanding that any image processing technique may be used.

The term "network interface" or similar terms, refer to any mechanism for aiding communications between various nodes or locations in a network. A network interface may include, for example a bus, a modem, or any other input/output structure. A network interface may permit a connection to any network capable of being connected to an input and/or output module located within at least one or more of the following exemplary networks: an Ethernet network, an Internet Protocol network, a telephone network, a radio network, a cellular network, or any mechanism for permitting communication between two or more modes or remote locations. In some invention embodiments, a network interface might also included a user interface.

The term "user interface" may include at least one component such as a keyboard, key pad, mouse, track ball, telephone, scanner, microphone, touch screen, web cam, interactive voice response system (IVR), voice recognition system or any other suitable input mechanism for conveying information. A user interface may also include an input port connected by a wired, optical, or wireless connection for electromagnetic transmissions. In some embodiments, a user interface may include connections to other computer systems to receive the input commands and data therefrom. User interface may further include a data reading device such as a disk drive for receiving input data from and writing data to storage media such as magnetic and optical disks.

As used herein terms such as "external body condition", "skin condition", and "actual condition" refer to conditions of at least one of the skin, teeth, hair, eyebrows, eyelashes, body hair, facial hair, fingernails, and/or toenails, or any other externality. Examples of skin conditions may include elasticity, dryness, cellulitis, sweating, aging, wrinkles, , exfoliation, desquamation, homogeneity of color, creases, liver spots, clarity, lines, micro-circulation, shininess, softness, smoothness, tone, texture, matitty, hydration, sag, suppleness, stress, springiness, firmness, sebum production, cleanliness, translucency, luminosity, irritation, redness, vasocolation, vasomotion, vasodilation, vasoconstriction, pigmentation, freckles, blemishes, oiliness, pore distribution, pore size, moles, birthmarks, acne, blackheads, whiteheads, pockmarks, warts, pustules, boils, blisters, marks, smudges, specks, and other characteristics associated with the subject's skin. Examples of hair conditions may include keratin plug, length, dryness, oiliness, dandruff, pigmentation, thickness, density, root conditions, split ends, hair loss, hair thinning, scales, staging, cleanliness and other properties related to the subject's hair. Examples of fingernail and toenail conditions may include, split nails, delaminating, , brilliancy, lines, spots, coloration, gloss, strength, brittleness, thickness, hangnail, length, , and other characteristics related to the subject's nails. Other conditions may include, for example, size and proportion of facial features, teeth discoloration, and any other aesthetic-related conditions of the user.

"Enabling", "facilitating", and "causing" an action refer to one or more of a direct act of performing the action, and any indirect act of encouraging or being an accessory to the action. Thus, the terms include partnering or cooperating with an entity who performs the action and/or referring commerce to or having commerce referred from an entity who performs the action. Other examples of indirect activity encompassed within the definitions of "enabling", "facilitating", and "causing" may include providing a subject with one or more of tools to knowingly aid in performing the action, providing instructions on how to perform the action, providing prompts or cues to perform the action, or expressly encouraging performance of the action. Indirect activity may also include cooperating with an entity who either directly performs the action or who helps another perform the action. Tools may include software, hardware, or access (either directly, through hyperlink, or some other type of cooperation or partnering) to a network location (e.g., web site) providing tools to aid in performing the action. Thus, phrases such as "enabling access" and "enabling display" do not necessary require that the actor actually access or display anything. For example, the actor may perform the enabling function by affiliating with an entity who performs the action, or by providing instructions, tools, or encouragement for another to do the accessing and displaying.

Forms of the word "displaying" and like terms may also include indirect acts such as providing content for transmission over a network to a display device, regardless of whether the display device is in the custody or control of the sender. Any entity in a chain of delivering information for display performs an act of "displaying", as the term is used herein.

Likewise, the term "providing" includes direct and indirect activities. For example, providing access to a computer program may include at least one of providing access over a network to the computer program, and creating or distributing to the subject a computer program configured to run on the subject's workstation or computer. For example, a first party may direct network traffic to (either through electronic links or through encouragement to visit) a server or web site run by a second party. If the second party maintains a particular piece of software thereon, then it is to be understood that within the meaning of "providing access" as used herein, the first party is said to provide access to the particular software. Or if the first party directs a subject to a second party who in turn ships the particular software to the user, the first party is said to provide the user with access to the particular software. (Of course, in both of the above instances, the second party would also be providing access within the meaning of the phrase as used herein.) "Receiving" may include at least one of acquisition via a network, via verbally communication, via electronic transmission, via telephone transmission, in hard-copy form, or through any other mechanism enabling reception. In addition, "receiving" may occur either directly or indirectly. For example, receipt may occur through a third party acting on another party's behalf, as an agent of another, or in concert with another. Regardless, all such indirect and direct actions are intended to be covered by the term "receiving" as used herein. A received request, for example, may take one of many forms. It may simply be a checked box, clicked button, submitted form or oral affirmation. Or it might be a typed or handwritten textual request. Receiving may occur through an on-line interest form, e-mail, facsimile, telephone, interactive voice response system, or file transfer protocol transmitted electronically over a network at a web site, an internet protocol address, or a network account. A request may be received from a subject for whom information is sought, or an entity acting on the subject's behalf. "Receiving" may involve receipt directly or indirectly through one or more networks and/or storage mediums. Receipt may occur physically such as in hard copy form, via mail delivery or other courier delivery.

Forms of the word "maintain" are used broadly to include gathering, storing, accessing, providing access to, or making something available for access, either directly or indirectly. For example, those who maintain information include entities who provide a link to a site of a third party where the information is stored.

Consistent with the concepts set forth above, all other recited actions such as, for example, obtaining, determining, generating, selecting, applying, simulating, presenting, etc, are inclusive of direct and indirect actions. Thus, for purposes of interpreting the following claims, an entity performs a recited action through either direct or indirect activity. Further examples of indirect activity include sending signals, providing software, providing instructions, cooperating with an entity to have the entity perform the action, outsourcing direct or indirect actions, or serving in any way as an accessory to the specified action.

The term "product" is used to generically refer to tangible merchandise, goods, services, and actions performed. A "beauty product," "beauty care product," "cosmetic product" or similar terms, refer to products (as defined above) for effecting one or more external body conditions, such as conditions of the skin, hair and nails. Examples of tangible merchandise forms of beauty products include cosmetic goods, such as treatment products, personal cleansing products, and makeup products, in any form (e.g., ointments, creams, gels, sprays, supplement, ingesta, inhalants, lotions, cakes, liquids, and powders.)

Examples of services forms of beauty products include hair styling, hair cutting, hair coloring, hair removal, skin treatment, make-up application, and any other offering for aesthetic enhancement. Examples of other actions performed include massages, facial rubs, deep cleansings, applications of beauty product, exercise, therapy, or any other action effecting the external body condition whether performed by a professional, the subject, or an acquaintance of the subject.

The following is exemplary and non-exhaustive listing of a few beauty products- scrubs, rinses, washes, moisturizers, wrinkle removers, exfoliates, toners, cleansers, conditioners, shampoos, cuticle creams, oils, and anti-fungal substances, anti-aging products, anti-wrinkle products, anti-freckle products, skin conditioners, skin toners, skin coloring agents, tanners, bronzers, skin lighteners, hair coloring, hair cleansing, hair styling, elasticity enhancing products, agents, blushes, mascaras, eyeliners, lip liners, lipsticks, lip glosses, eyebrow liners, eye shadows, nail polishes, foundations, concealers, dental whitening products, cellulite reduction products, hair straighteners and curlers, and weight reduction products. A beauty care treatment regimen may involve the administration of one or more products, as defined above.

The terms "beauty advice", "beauty guidance", and similar terms are used interchangeably to refer to the provision of beauty related information to a subject. Advice or guidance includes one or more of beauty product recommendations (e.g., cosmetic product recommendations for products to treat conditions the subject is prompted to evaluate), remedial measures, preventative measures, predictions, prognoses, price and availability information, application and use information, suggestions for complementary products, lifestyle or dietary recommendations, or any other information intended to aid a subject in a course of future conduct, to aid a subject in understanding past occurrences, to reflect information about some future occurrences related to the subject's beauty or to aid a subject in understanding beauty products, as defined above.

The terms "beauty product" and "cosmetic product" refer to a product as defined in the Council Directive 93/35/EEC of 14 June 1993.

The term "network" may include a public network such as the Internet or a telephony network, a private network, a virtual private network, or any other mechanism for enabling communication between two or more nodes or locations. The network may include one or more of wired and wireless connections. Wireless communications may include radio transmission via the airwaves, however, those of ordinary skill in the art will appreciate that various other communication techniques can be used to provide wireless transmission including infrared line of sight, cellular, microwave, satellite, blue-tooth packet radio and spread spectrum radio. Wireless data may include, but is not limited to, paging, text messaging, e-mail, Internet access and other specialized data applications specifically excluding or including voice transmission.

In some instances consistent with the invention, a network may include a courier network (e.g. postal service, United Parcel Service, Federal Express, etc.). Other types of networks that are to be considered within the scope of the invention include local area networks, metropolitan area networks, wide area networks, ad hoc networks, or any mechanism for facilitating communication between two nodes or remote locations.

"Artificial intelligence" (Al) is used herein to broadly describe any computationally intelligent systems that combine knowledge, techniques, and methodologies. An Al engine may be any system configured to apply knowledge and that can adapt itself and learn to do better in changing environments. Thus, the Al engine may employ any one or combination of the following computational techniques: neural network, constraint program, fuzzy logic, classification, conventional artificial intelligence, symbolic manipulation, fuzzy set theory, evolutionary computation, cybernetics, data mining, approximate reasoning, derivative-free optimization, decision trees, or soft computing. Employing any computationally intelligent techniques, the Al engine may learn to adapt to unknown or changing environment for better performance. Al engines may be implemented or provided with a wide variety of components or systems, including one or more of the following: central processing units, co-processors, memories, registers, or other data processing devices and subsystems.

Al engines may be trained based on input such as product information, expert advice, user profile, or data based on sensory perceptions. Using input an Al engine may implement an iterative training process. Training may be based on a wide variety of learning rules or training algorithms. For example, the learning rules may include one or more of the following: back-propagation, real-time recurrent learning, pattern-by-pattern learning, supervised learning, interpolation, weighted sum, reinforced learning, temporal difference learning, unsupervised learning, or recording learning. As a result of the training, Al engine may learn to modify its behavior in response to its environment, and obtain knowledge. Knowledge may represent any information upon which Al engine may determine an appropriate response to new data or situations. Knowledge may represent, for example, relationship information between two or more products. Knowledge may be stored in any form at any convenient location, such as a database.

Since Al engine may learn to modify its behavior, information describing relationships for a universe of all combinations of products may not need to be maintained by the Al engine or any other component of the system.

"Personal information", "subject specific information", "user specific information", "user profile", "personal characteristics", "personal attributes", "profile information", and like terms (collectively referred to in this section as "personal information") may broadly encompass any information about the subject or user. Such information may, for example, fall within categories such as physical characteristics, fashion preferences, demographics, nutritional information, cosmetic usage information, medical history information, environmental information, beauty product usage information, lifestyle, and may include information such as name; age; birth date; height; weight; ethnicity; eating habits; vacation patterns; geographic location of the individual's residence, location, or work; work habits; sleep habits; toiletries used; exercise habits; relaxation habits; beauty care habits; smoking and drinking habits; sun exposure habits; use of sunscreen; propensity to tan; number of sunburns and serious sunburns; dietary restrictions; dietary supplements or vitamins used; diagnosed conditions affecting the external body, such as melanoma; an image, such as a picture or a multimedia file of the subject; facial feature characteristics; family history information such as physical characteristics information about relatives of the subject (e.g., premature balding, graying, wrinkles, etc.); external body condition (as defined previously); color preferences, clothing style preferences, travel habits; entertainment preferences; fitness information; adverse reactions to products, compounds, or elements (e.g., sun exposure); body chemistry, use of prior beauty care products and their effectiveness; purchasing, shopping, and browsing habits; hobbies; marital status; whether the subject is a parent; country of residence; region of residence; birth country and region; religious affiliation; political affiliation; whether the subject is an urban dweller suburban dweller or rural area dweller; size of urban area in which the subject lives; whether the subject is retired; annual income, sexual preference, or any other information reflecting habits, preferences, or affiliations of the subject.

Personal information may also include information electronically gleaned by tracking the subject's electronic browsing or purchasing habits, or as the result of cookies maintained on the subject's computer, responses to surveys, or any other mechanism providing information related to the subject. In addition, personal information may be gathered through non-electronic mechanisms such as hard copy surveys, personal interviews, or consumer preference polls. "Complementary" and "complementary product" refers to one or more of physical, physiological, biologically, and aesthetic compatibility. A product may be complementary with one or more of another product, a group of products, or a subject. In that latter instance, whether a product is considered "complementary" may be a function of personal information of the subject. Thus, for example a product may be complementary if it is unlikely to cause an adverse allergic reaction; if it physically blends well with another product; or if it is aesthetically consistent with the subject or one or more other products. Aesthetic compatibly may refer to the fact that two products are aesthetically appealing (or do not clash) when worn together. The identification of a complementary product may also be based on product characteristics, user preferences, survey data, or expert advice.

As used herein, the words "may" and "may be" are to be interpreted in an open-ended, non-restrictive manner. At minimum, "may" and "may be" are to be interpreted as definitively including structure or acts recited. Further, the word "or" is to be interpreted in the conjunctive and the disjunctive.

All medical or therapeutical methods for treatment of the human or animal body and diagnostic method practised on the human or animal body contrary to Article 52(4) EPC depart from the scope of the present application.

While flow charts presented herein illustrate a series of sequential blocks for exemplary purposes, the order of blocks is not critical to the invention in its broadest sense. Further, blocks may be omitted and others added without departing from the spirit of the invention. Also, the invention may include combinations of features described in connection with differing embodiments.

Although a focus of the disclosure may be on server-side methods, it is nevertheless to be understood that the invention includes corresponding client-side methods, software, articles of manufacture, and computer readable media, and that computer readable media can be used to store instructions for some or all of the methods described herein. Further, it is to be understood that disclosed structures define means for implementing the functionality described herein, and that the invention includes such means for performing the disclosed functions.

In the foregoing Description of Exemplary Embodiments, various features are grouped together in a single embodiment for purposes of streamlining the disclosure. This method of disclosure is not to be interpreted as reflecting an intention that the claimed invention requires more features than are expressly recited in each claim. Rather, as the following claims reflect, inventive aspects lie in less than all features of a single foregoing disclosed embodiment. Thus, the following claims are hereby incorporated into this Description of the Exemplary Embodiments, with each claim standing on its own as a separate embodiment of the invention.

## Claims

1. System for tracking progress of a beauty treatment regimen, the system comprising:
means for prescribing the beauty treatment regimen to an individual, the prescribing means being suitable for altering a condition of at least one of skin, hair, teeth and nails, wherein a typical individual undergoing the beauty treatment regimen exhibits at least one indicia of progress, the indicia of progress being substantially imperceptible to a typical human eye in at least one stage of the beauty treatment regimen;
means for tracking the indicia of progress with respect to the individual; and
means for advising the individual about positive progress in the beauty treatment regimen.

2. System according to the preceding claim, further including means for encouraging the individual to continue the beauty treatment regimen regardless of whether the positive progress is substantially imperceptible to the typical human eye.

3. System according to any one of the preceding claims, further including means for advising the individual about a predicted synergy of an additional treatment for use with the prescribed beauty treatment regimen.

4. System according to the preceding claim, wherein the prescribed beauty treatment regimen includes a wrinkle treatment and the additional treatment is an elasticity treatment.

5. System according to any one of the two preceding claims, wherein the advising means advises the individual about a predicted synergy occurs when the indicia of progress is below a desired threshold.

6. System according to any one of the preceding claims, further including means for altering the beauty treatment regimen when the indicia of progress is below a desired threshold.

7. System according to any one of the preceding claims, wherein the tracking means includes means for collecting information.

8. System according to any one of the preceding claims, wherein the tracking means includes means for directing the individual to conduct at least a portion of at least one self-test, and directing the individual to record a result of the at least a portion of at least one self-test.

9. System according to the preceding claim, wherein the at least one self-test is a test of at least one of elasticity, oiliness, pH, texture, color, and pigmentation of at least one of the individual's skin, hair, and nails.

10. System according to any one of the preceding claims, wherein the tracking means is configured to collect a series of data points to form a curve and calculates an area under the curve.

11. System according to any one of the preceding claims, wherein the beauty treatment regimen is at least one of a treatment for wrinkle reduction, anti-aging, elasticity improvement, enhanced coloration, improved clarity, skin blemish removal, and freckle reduction.

12. System according to any one of the preceding claims, further including means for conducting over a network at least a portion of the beauty analysis on the individual prior to prescribing.

13. System according to the preceding claim, wherein during the beauty analysis, the individual is instructed to conduct at least a portion of at least one self-test, and to electronically report a result of the at least a portion of at least one self-test.

14. System according to the preceding claim, further including means for enabling electronic reporting of beauty information, and wherein during the beauty analysis, the individual is instructed to conduct at least a portion of at least one self-test, and to electronically report a result of the at least a portion of at least one self-test using the electronic reporting means.

15. System according to any one of the preceding claims, wherein at least one of the prescribing, tracking and advising means are computer-related products provided to the individual.

16. System according to any one of the preceding claims, wherein the at least a one of the prescribing, tracking and advising means includes a network.

17. System according to any one of the preceding claims, further comprising:
information collection means for capturing at least two images of portions of the individual's body;
means for comparing the at least two images of portions of the individual's body to ascertain treatment effectiveness; and
means for advising the individual of information reflective of the treatment effectiveness.

18. System according to the preceding claim, wherein the comparing means is configured to quantify differences in wrinkles between the at least two images.

19. System according to any one of the two preceding claims, wherein the comparing means is configured to quantify differences in coloration between the at least two images.

20. System according to any one of claims 17 to 19, wherein the comparing means is configured to quantify differences in at least one of skin blemishes and freckles between the at least two images.

21. System according to any one of claims 17 to 20, wherein the tracking means includes means for reporting test results to a laboratory.

22. A method of tracking progress of a beauty treatment regimen, the method comprising:
prescribing the beauty treatment regimen to an individual, the regimen for altering a condition of at least one of skin, hair, teeth and nails, wherein a typical individual undergoing the beauty treatment regimen exhibits at least one indicia of progress, the indicia of progress being substantially imperceptible to a typical human eye in at least one stage of the beauty treatment regimen;
tracking the indicia of progress with respect to the individual; and
advising the individual about positive progress in the beauty treatment regimen.

23. Method according to the preceding claim, further including encouraging the individual to continue the beauty treatment regimen regardless of whether the positive progress is substantially imperceptible to the typical human eye.

24. Method according to any one of the two preceding claims, further including advising the individual about a synergy of an additional treatment for use with the prescribed beauty treatment regimen.

25. Method according to the preceding claim, wherein the prescribed beauty treatment regimen includes a wrinkle treatment and the additional treatment is an elasticity treatment.

26. Method according to any one of the two preceding claims, wherein advising the individual about a synergy occurs when the indicia of progress is below a desired threshold.

27. Method according to any one of claims 22 to 26, further including altering the beauty treatment regimen when the indicia of progress is below a desired threshold.

28. Method according to any one of claims 22 to 27 claim, wherein tracking involves the use of an image capture device.

29. Method according to any one of claims 22 to 28, wherein tracking includes auto-evaluation by the individual.

30. Method according to any one of claims 22 to 29, wherein tracking includes directing the individual to conduct at least a portion of at least one self-test, and directing the individual to record a result of the at least a portion of at least one self-test.

31. Method according to the preceding claim, wherein the at least one self-test is a test of at least one of elasticity, oiliness, pH, texture, color, and pigmentation of at least one of the individual's skin, hair, teeth, and nails.

32. Method according to any one of claims 22 to 31, wherein tracking includes directing physical test materials to be used on the individual, and directing transmission of used test materials to a laboratory for analysis.

33. Method according to any one of claims 22 to 32 wherein tracking includes collecting a series of data points to form a curve and calculating an area under the curve.

34. Method according to any one of claims 22 to 33, wherein the beauty treatment regimen includes at least one of a treatment for wrinkle reduction, anti-aging, elasticity improvement, enhanced coloration, improved clarity, skin blemish removal, and freckle reduction.

35. Method according to any one of claims 22 to 34, further including, conducting over a network at least a portion of a beauty analysis on the individual prior to prescribing.

36. Method according to the preceding claim, wherein during the beauty analysis, the individual is instructed to conduct at least a portion of at least one self-test, and to electronically report a result of the at least a portion of at least one self-test.

37. Method according to any one of claims 22 to 36, wherein prescribing, tracking and advising are accomplished by providing the individual with computer-related product, and wherein the computer-related product is configured to perform at least a portion of the prescribing, tracking, and advising.

38. Method according to any one of claims 22 to 37, wherein the at least a portion of prescribing, tracking and advising is accomplished using a network that is accessible by the individual.

39. Method according to any one of claims 22 to 38, wherein tracking includes obtaining a body condition image and magnifying the image.

40. Method according to any one of claims 22 to 39, wherein tracking includes directing application of powder to the individual.

41. Method according to any one of claims 22 to 40, wherein tracking includes directing illumination of the individual's skin with a Woods lamp.

42. Method according to any one of claims 22 to 41, further comprising:
capturing at least two images of portions of the individual's body using an image capture device;
comparing, using image processing, at least two images of portions of the individual's body to ascertain treatment effectiveness; and
advising the individual of information reflective of the treatment effectiveness.

43. Method according to any one of claims 22 to 41, comprising:
capturing a first image of a portion of the individual's body;
detecting skin defects by image processing on the first image;
quantifying the skin defects detected on the first image;
capturing a second image of a portion of the individual's body;
detecting skin defects by image processing on the second image;
comparing, using image processing, the skin defects of the first and second images to ascertain treatment effectiveness; and
advising the individual of information reflective of the treatment effectiveness.

44. Method according to the preceding claim, wherein the skin defects are detected using color analysis.

45. Method according to claim 43, wherein the skin defects are detected using shape analysis.

46. Method according to any one of claims 22 to 45, wherein during image processing, wrinkles in a first image of portions of the individual's body are quantified, and compared with wrinkles quantified in a second image of portions of the individual's body.

47. Method according to any one of claims 22 to 46, wherein during image processing, coloration in a first image of portions of the individual's body is quantified, and compared with coloration in a second image of portions of the individual's body.

48. Method according to any one of claims 22 to 47, wherein during image processing, at least one of skin blemishes and freckles in a first image of portions of the individual's body are quantified, and compared with at least one of skin blemishes and freckles in a second image of portions of the individual's body.

49. Method according to any one of claims 22 to 48, wherein tracking includes obtaining a body condition image and magnifying the image.

50. Method according to any one of claims 22 to 49, wherein tracking includes directing application of powder to the individual.

51. Method according to any one of claims 22 to 50, wherein tracking includes directing illumination of the individual's skin with a Woods lamp.

52. Method according to any one of claims 22 to 51, wherein altering includes adjusting a catalyst level at a skin surface of the subject.

53. Method according to any one of claims 22 to 52, wherein altering includes adjusting a phosphate level at a skin surface of the subject.

54. A method of performing a skin analysis, the method comprising:
altering a chemical level in a subject's body;
prescribing a beauty treatment regimen to the subject;
receiving measurements reflecting a period of time required for the chemical level to cross a threshold level;
determining from at least the measured period of time, a projected effectiveness of the prescribed beauty treatment regimen; and
advising the subject of the projected effectiveness before an actual effectiveness is perceptible to the subject.

55. A system of tracking progress of a beauty treatment regimen, the system comprising:
means for prescribing the beauty treatment regimen to an individual, the prescribing means for altering a condition of at least one of skin, hair, teeth and nails, and wherein a typical individual undergoing the beauty treatment regimen exhibits at least one indicia of progress, the indicia being substantially imperceptible to a typical human eye in at least one stage of the beauty treatment regimen;
means for tracking the indicia of progress with respect to the individual; and
information collection means for capturing at least two images of portions of the individual's body;
means for comparing the at least two images of portions of the individual's body to ascertain treatment effectiveness; and
means for advising the individual of information reflective of the treatment effectiveness.

56. A method of tracking progress of a beauty treatment regimen, the method comprising:
prescribing the beauty treatment regimen to an individual for altering a condition of at least one of skin, hair, teeth and nails, wherein a typical individual undergoing the beauty treatment regimen exhibits at least one indicia of progress, the indicia being substantially imperceptible to a typical human eye in at least one stage of the beauty treatment regimen;
tracking the indicia of progress with respect to the individual;
capturing at least two images of portions of the individual's body using an image capture device;
comparing, using image processing, at least two images of portions of the individual's body to ascertain treatment effectiveness; and
advising the individual of information reflective of the treatment effectiveness.
